# EUROPEAN PATENT SPECIFICATION

(11) **EP 4 136 053 B1**
(45) Date of publication and mention of the grant of the patent: **31.01.2024**
(21) Application number: 21717080.2
(22) Date of filing: 07.04.2021
(51) Int. Cl.: C01B 3/02, C01C 1/04, C07C 273/04

(54) **REVAMPING OF AMMONIA-UREA PLANTS**
NACHRÜSTUNG VON AMMONIAKHARNSTOFFANLAGEN
MODERNISATION D'UNE INSTALLATION D'AMMONIAC-URÉE

(30) Priority: 17.04.2020 EP 20170057
(43) Date of publication of application: 22.02.2023
(73) Proprietor: CASALE SA, 6900 Lugano (CH)
(72) Inventor: IOCCHI, Filippo, 6900 Lugano (CH); MARRONE, Leonardo, 21020 Mercallo (VA) (IT)
(74) Representative: M. Zardi & Co S.A.
(86) International application number: PCT/EP2021/059098
(87) International publication number: WO 2021/209299

(56) References cited:
- US-A1- 2017 260 060
- US-A1- 2020 055 738
- Jj De Wit: "Advantages of Electric Motor in Ammonia/Urea Complexes", Ammonia Plant Safety; Meeting: 41st Annual Safety in Ammonia Plants and Related Facilities Symposium, 1 January 1996 (1996-01-01), pages 157-165, XP055729771, Retrieved from the Internet: URL:http://www.iffcokandla.in/data/polopol y_fs/1.2497153.1437770447|/fileserver/file /514268/filename/Aiche-37-018.pdf [retrieved on 2020-09-11]
- "Meeting demands for today and beyond", NITROGEN + SYNGAS, BRITISH SULPHUR PUBLISHING, LONDON, GB, no. 319, 1 September 2012 (2012-09-01), pages 44-51, XP001579730, ISSN: 1750-6891

## Description

### Field of the invention

The present invention relates to the field of ammonia-urea integrated plants. Particularly, the invention relates to revamping of ammonia-urea plants and efficient integration of the steam networks of the ammonia section and urea section.

### Prior art

Urea is produced industrially by reacting ammonia and carbon dioxide. The production of ammonia typically involves the generation of a makeup synthesis gas (syngas) from reforming, purification of the syngas and catalytic conversion of the syngas to produce ammonia. The purification of the syngas normally includes shift conversion of carbon monoxide CO to carbon dioxide CO2 and removal of CO2.

The production of ammonia and urea, therefore, can be advantageously integrated. A urea plant coupled to an ammonia plant may use as source material all or part of the ammonia produced in the ammonia plant, as well as the CO2 removed from the syngas during purification.

The term ammonia-urea plant denotes an installation including an ammonia section where ammonia is produced and a urea section where at least part of the ammonia produced in the ammonia section is used for the synthesis of urea. Preferably, also part or all of the carbon dioxide removed from the syngas in the ammonia section is used for the synthesis of urea. An input of fresh carbon dioxide may also be provided if necessary.

The ammonia section generally includes the front-end for generation of syngas by steam reforming and its purification, and a synthesis section where the syngas is catalytically reacted to produce ammonia. The front-end in turn includes a reforming section and a purification section. The reforming section may have different arrangements, e.g. including a primary reformer and a secondary reformer, an autothermal reformer, and others.

The purification section generally includes one or more shift converters to convert CO into CO2 and a CO2 removal section. The CO2 removal section may use various CO2 separation techniques such as, for example, amine-based absorption or other like temperature-swing adsorption (TSA) or a pressure-swing adsorption (PSA).

The purified syngas shall have a suitable nitrogen to hydrogen ratio for the synthesis of ammonia. If necessary, nitrogen may be added to the syngas in order to reach the target ratio.

The synthesis of ammonia is performed at a high pressure which means that the syngas needs be compressed. The compressor is typically a multi-stage compressor driven by a steam turbine.

The starting material for the steam reforming may be a suitable hydrocarbon, such as natural gas.

The urea section may use any of the known techniques for the synthesis of urea but most preferably, urea is produced with a stripping process. The term stripping process identifies a process wherein the effluent of a urea synthesis reactor is processed in a stripper to remove unconverted ammonia and carbon dioxide.

In accordance with the stripping process, the urea section may include a high-pressure urea synthesis section and a recovery section. The high-pressure synthesis section operates at a pressure over 100 bar, typically 140 to 200 bar. The reagents need be compressed at such high pressure. Particularly the carbon dioxide, be it a fresh stream and/or the CO2 removed from the syngas, needs be compressed to the urea synthesis pressure. Like the ammonia syngas compressor, also the CO2 compressor is a large, multi-stage compressor typically driven by a steam turbine.

The recovery section has basically the function of remove and recover unconverted reagents contained in the urea-containing solution produced in the synthesis section and may operate at one or more lower pressure(s). For example a urea plant may include a low-pressure recovery section or a medium-pressure recovery section followed by a low-pressure recovery section.

The high pressure synthesis section includes at least a urea synthesis reactor, a high-pressure stripper and a high-pressure condenser. Optionally it may include a scrubber for vapours extracted from the reactor.

The urea synthesis rector produces a reactor effluent (urea-containing aqueous solution) which contains a significant amount of unconverted ammonia and carbon dioxide, mostly in the form of ammonium carbamate. In the stripper, the effluent of the reactor is heated and optionally contacted with a stripping gas (e.g. carbon dioxide) to decompose the ammonium carbamate into ammonia and carbon dioxide and then remove the ammonia and carbon dioxide in a gaseous phase. Typically, the stripper is a steam-heated shell-and-tube apparatus.

The stripper vapours, containing the ammonia and CO2 removed from the reactor effluent, are condensed in the high-pressure condenser to obtain a condensate which is returned to the reactor.

The urea-containing solution obtained from the stripper is further processed in the recovery section. The processing in a recovery section includes heating the solution in a carbamate decomposer and condensation of the so obtained vapours, obtaining a carbamate solution which can be returned to the high-pressure section, for example introduced in the high-pressure condenser.

The most common urea stripping processes are: the CO2 stripping process, the self-stripping process and the ammonia stripping process. In the CO2 stripping process (originally developed by Stamicarbon), the stripping of the urea-containing solution is performed with the aid of gaseous CO2 as stripping agent and the subsequent recovery is typically performed in a single recovery section at low pressure. In a self-stripping process the stripping is performed without a stripping agent and recovery is performed in a medium-pressure section followed by a low-pressure section. The ammonia-stripping process is a variant of the self-stripping process where gaseous ammonia is introduced in the stripper to act as stripping agent and recovery is still performed at medium pressure and low pressure.

Both the ammonia section and the urea section include a steam network. The steam network denotes the assembly of various steam producers and steam users and the headers that connect them.

A steam producer is typically a piece of equipment where heat is removed from a process stream. Examples are a gas cooler, e.g. to cool the hot gas form reforming before the shift conversion, or a condenser (e.g. the high-pressure condenser of the urea section) where condensation heat is transferred to cooling water and steam is generated.

A steam user may be a steam expander, such as the steam turbines of the syngas compressor and CO2 compressor, or a piece of equipment which requires heat. For example the CO2 removal section of the ammonia front-end may require heat for regeneration of a CO2 absorbing solution. Other examples of steam users include e.g. the high-pressure stripper and the carbamate decomposers in the recovery section. Steam users can be classified as power users (PUs) when the steam is used to generate power (e.g. turbines) and thermal users (TUs) where steam is used to provide a desired heat input to the process (e.g. a stripper or decomposer).

A steam producer may generate steam at a high pressure or low pressure depending on the temperature of the heat source, e.g. of the process stream from which heat is removed. Generally, the amount of HP steam that can be produced by heat recovery is limited, and HP steam may be produced by auxiliary boilers when needed.

In the prior art of ammonia-urea plants, each of the ammonia section and urea section has its own steam network. Some high-pressure steam normally produced in an auxiliary boiler, is typically exported from the steam network of the ammonia section to the steam network of the urea section, to power the steam turbine of the CO2 compressor. A medium-pressure (about 23 bar) steam extracted from said turbine is typically used to heat the high-pressure stripper (i.e. to provide heat for decomposition of carbamate). Low pressure steam for other thermal users of the urea section is produced by the carbamate condenser of the synthesis loop, typically at a pressure of around 3.5 bar.

The medium-pressure recovery section, when provided, includes typically a carbamate decomposer which requires steam at a pressure higher than the pressure of the steam produced in the carbamate condenser, for example around 5.5 bar. There is no steam producer in the urea section which is able to produce steam directly at such pressure, therefore this steam is normally produce by mixing a portion of the medium-pressure steam taken from the above mentioned turbine of the CO2 compressor with a portion of the low-pressure steam produced in the carbamate condenser in an ejector. However ejectors are very inefficient as known in the art.

The ammonia section may also produce an excess of low-pressure steam, possibly as a consequence of a revamping. For example, an excess of LP steam may be the result of installation of a backpressure turbine to drive auxiliary equipment or of modernization of existing thermal users, for example modernization of a CO2 recovery section to reduce its thermal consumption.

In such a case, when an additional LP steam is available, the prior art suggests its exploitation in the ammonia section by installing suitable users like vapour absorption cooling systems or chemical heat pumps, which are however expensive and inefficient.

Jj De Wit: "Advantages of Electric Motor in Ammonia/Urea Complexes",Ammonia Plant Safety; Meeting: 41st Annual Safety in Ammonia Plants and Related Facilities Symposium, 1 January 1996 (1996-01-01), pages 157-165, XP055729771, discloses different revamping approaches for ammonia-urea plants including export of steam. In one example excess low pressure steam is exported from the urea section to the ammonia section.

Also, the above mentioned mixing of medium-pressure steam with LP steam is inefficient from the energetic point of view. An inefficient recovery of thermal energy increases the fuel consumption of the plant. The fuel may include for example fuel of a fired furnace used for primary reforming and fuel of auxiliary boilers for the production of steam.

There is therefore the need to provide a more efficient use of steam and integration between the steam networks in the ammonia-urea plants, particularly when a plant is revamped and more LP steam is available in the ammonia section as a result of the revamping.

### Summary of the invention

An aspect of the invention is a method of revamping an ammonia-urea plant according to claim 1.

The revamping includes the the provision of an export of low pressure steam from the ammonia section to the urea section, for use in at least one steam user in the urea section. Said low pressure stream which is transferred (exported) from the ammonia section to the urea section has a pressure not greater than 6 bar preferably 2 to 6 bar. Even more preferably said steam is at a pressure of 3 bar to 4 bar. This is the pressure at the thermal user in the urea plant.

This extra amount of low pressure steam for the urea section can be exploited in different ways in accordance with the features of the urea section and kind of urea synthesis process. The urea section may be modified in view of the extra amount of LP steam coming from the ammonia section.

Preferably the urea section is modified to accommodate said low pressure steam exported from the ammonia section to the urea section. The consumption of medium-pressure steam may be consequently reduced.

In some embodiments the urea section may be modified to produce internally steam at a pressure greater than the pressure of the steam imported from the ammonia section, for a particular steam user like for example a carbamate decomposer of a medium-pressure recovery section. The imported steam is then used to balance the request of low-pressure steam. Said internal production of steam may replace an inefficient mixing of MP steam and LP steam, to the advantage of the overall fuel consumption.

In an embodiment, the method includes that the condensation stage of the urea section is modified for direct production of steam for use in a MP carbamate decomposition in a medium-pressure recovery section. Some of the heat that can be recovered from the stripper vapours is used, in the modified condensation stage, to produce said steam for the MP carbamate decomposition. As a consequence of this, the urea section will produce less LP steam and the need of LP steam in the urea section is then balanced by the LP steam imported from the ammonia section.

In a variant of this embodiment, some steam produced in the carbamate condenser of the synthesis section may be compressed to a higher pressure required by a steam user.

In another embodiment, a CO2 stripping urea section can be modified by reducing the heat transferred to the high-pressure stripper. In terms of the urea process, this means that part of the duty of the stripper is shifted to the low-pressure recovery section and the stripper consumes less steam for its heating. As the stripper requires a valuable steam at medium pressure, this is a considerable advantage from the energetic point of view. The reduced duty of the stripper causes that less LP steam can be produced in the carbamate condenser of the synthesis section, however the missing LP steam is provided by the LP steam import from the ammonia section.

Therefore the main advantage of the invention is a better recovery and use of thermal energy within the ammonia-urea process, which ultimately results in reducing the fuel consumption of the overall process. For example the invention may reduce the need of MP steam extraction from the turbine of the CO2 compressor. A reduced MP turbine extraction results in a reduced input of HP steam of the turbine which is produced at the expense of fuel, and therefore is a fuel saving.

Some preferred embodiments of the invention are given in the dependent claims. Another aspect of the invention is a process according to the claims.

### Preferred embodiments

The term of low pressure (LP), when referring to steam, denotes a pressure of less than 6 bar and preferably 2 to 6 bar. The term medium-low pressure (MLP) may be used to denote a pressure in the upper range or slightly above the low-pressure, typically of 4 to 10 bar. The term medium pressure (MP) typically denotes a pressure of 15 to 40 bar. The term high pressure (HP) typically denotes a pressure well above 40 bar, for example above 90 bar.

It should be noted that the terms of low-pressure, medium-pressure and high-pressure may also refer to the pressure in the process side. For example the high-pressure carbamate condenser of the urea synthesis section has a high pressure (generally greater than 100 bar) in the process side but the condensation heat removed from the stripper vapours has a low temperature and therefore the condenser produces steam at low pressure, typically of 3.5 bar. Also the terms of low-pressure recovery section and medium-pressure recovery section refer to the pressure in the process side.

In absence of a different indication all pressures are relative to the atmospheric pressure (bar rel). The atmospheric pressure is understood as 101325 Pa.

The urea section may include a steam user of steam at a second pressure greater than the pressure of said low pressure steam transferred from the ammonia section to the urea section. This is typically the case of a urea section including a medium pressure recovery section, where decomposition of carbamate at medium pressure requires steam at around 6 bar while the imported steam is at about 3.5 bar.

In such a case the imported steam cannot be used as such in this steam user. The method may include modifying the urea section to produce steam at said second pressure for said user and balancing the input of low-pressure steam to the urea section with the low-pressure steam transferred from the ammonia section to the urea section.

The steam at said second pressure may be produced by modifying the condensation stage of the high-pressure urea synthesis section to use part of the condensation heat of the stripper vapours to produce said steam at said second pressure. This can be made, in a preferred embodiment, with installation of a second condenser. Said second condenser may be a heat exchanger fitted in the same pressure vessel of an existing condenser or can have a separate pressure vessel..

Said second condenser may be installed in series and upstream the first condenser, so that the stripper vapours coming from the stripper pass through the newly installed second condenser and then through the original first condenser.

Preferably, the first condenser and/or the second condenser are configured as shell-and-tube equipment. More preferably they are horizontally arranged vessels of the kettle type, where the stripper vapours flow in the tube side and cooling water is evaporated in the shell side.

A step of modifying the urea section to produce steam at said second pressure may also include installation of a steam compressor which is fed with steam at low pressure and delivers steam at said second pressure.

In the ammonia-urea plant, the steam at said second pressure for said user may be originally obtained by mixing medium-pressure steam with low-pressure steam produced in the condensation stage. In such a case the method of the invention preferably includes that said mixing is discontinued.

The above embodiments including internal production in the urea section of steam at said second pressure (e.g. MLP steam around 6 bar) are typically applicable to a plant including a self-stripping urea section (e.g. Snamprogetti urea section) because this kind of urea process has a peculiar steam user requiring steam at about 6 bar, namely the carbamate decomposer of the medium-pressure recovery section.

The method invention is also applicable to plants having a urea section where urea is produced according to the CO2 stripping process. In such a case, to make use of the extra amount of LP steam from the ammonia section, the method may include reducing the amount of heat transferred to the stripper thus reducing also the amount of low pressure steam that can be produced in the high-pressure carbamate condenser. The missing production of low pressure steam in the urea section is then balanced with the low-pressure steam imported from the ammonia section.

The method of the invention may include that the ammonia section is modified to provide the low pressure steam which is required for balancing the low pressure steam input of the urea section. It can be seen that the modification of the ammonia section and of the urea section are synergetic. The modernization of the ammonia section may provide an extra amount of low-pressure steam, which may be caused by increased thermal efficiency of existing components and/or the provision of additional means for heat recovery. The modification of the urea section, at the same time provides that the urea section can import this extra amount of low-pressure steam, because the internal production of LP steam in the urea section is lowered to the advantage of a more valuable MP steam. From energetic and thermodynamic perspective, the plant is made more efficient.

In a preferred embodiment, at least part of the low pressure steam for balancing the input of the urea section is obtained from modernization of a CO2 recovery section in the front-end of the ammonia section. As stated above, the CO2 recovery section is a large user of low pressure steam, typically for regeneration of a CO2 solvent in an amine-based process or hot potassium carbamate process.

The ammonia-urea plant to be modified may originally include mixing of medium-pressure steam with low-pressure steam produced in the condensation stage and using the so obtained steam at a medium-low pressure to provide heat to a carbamate decomposer. In such a case, the method of the invention may include that said mixing is discontinued and said decomposer is heated with the MP stream produced in the modified condensation stage, for example in newly installed second condenser of the stripper vapours in the high-pressure synthesis section.

According to another preferred embodiment, the method includes that the steam to carbon ratio in the reforming process is lowered. This reduction of the steam to carbon ratio may be advantageous for the reforming process but reduces the amount of high pressure steam that can be produced by heat recovery. In this case, the overall reduction of high pressure steam consumption in the ammonia urea plant given by the invention compensates for this drawback of a lower heat recovery downstream the syngas generation units (e.g. primary and secondary reformers) due to a reduction of the steam to carbon ratio in the ammonia plant therefore enabling to maximise the benefits of lowered S/C in the plant.

More preferably, the steam to carbon ratio in the reforming process is lowered to a value in the range 2.6 to 2.0. If necessary the shift catalyst may be replace with a shift catalyst which is selected accordingly to sustain the modified low steam to carbon ratio. The reduction of the S/C in the ammonia plant leads to a trade-off between fuel consumption reduction, inert formation and the possibility to recover heat downstream the syngas generation units to operate the CO2 recovery section.

In a preferred embodiment, a modernization of the CO2 recovery section is performed, which reduces the regeneration heat required by the CO2 solvent through the installation of a new flash regeneration column. Said modernization, in synergy with the lower S/C, may reduce the specific regeneration heat required by CO2 recovery. In a preferred embodiment said specific regeneration heat is reduced down to less than 400 kcal/Nm3 of removed CO2.

A low S/C ratio may reduce the heat that can be recovered from the process gas in the ammonia section downstream the syngas generation units. This can be compensated, according to an embodiment of the invention, by a retrofit of large and inefficient consumers of steam, particularly of consumers of high-pressure steam. Said retrofit may include replacing a steam turbine drive with an electric motor drive. For example at least one of the boiler feed water pumps of the ammonia plant steam network is changed from a turbine driver to motor drive.

In a highly preferred embodiment, the ammonia section is revamped to operate the reforming process with a S/C ratio in the range of 2.6 to 2.0; the shift section is modified to operate with said reduced S/C ratio; at least one steam turbine driver is changed to a motor drive resulting in reduced steam consumption.

The above described embodiments can effectively increase the energy efficiency of the ammonia-urea plant, particularly with the adoption of a reduced S/C ratio in conjunction with the overall reduction of high-pressure steam consumption.

### Description of figures

Fig. 1 is a block scheme of a ammonia-urea plant of the prior art.
Fig. 2 is a scheme of the plant of Fig 1, modified in accordance with an embodiment of the invention.
Fig. 3 is a block scheme of a self-stripping urea section of the plant, according to a prior art.
Fig. 4 illustrates a scheme which is the result of a modification of the scheme of Fig. 3, after a revamping in accordance with an embodiment.
Fig. 5 illustrates a scheme a modification of the scheme of Fig. 3, after a revamping in accordance with another embodiment.
Fig. 6 illustrates an application of the invention to a plant where urea is produced with the CO2 stripping process.

### Detailed description of preferred embodiments

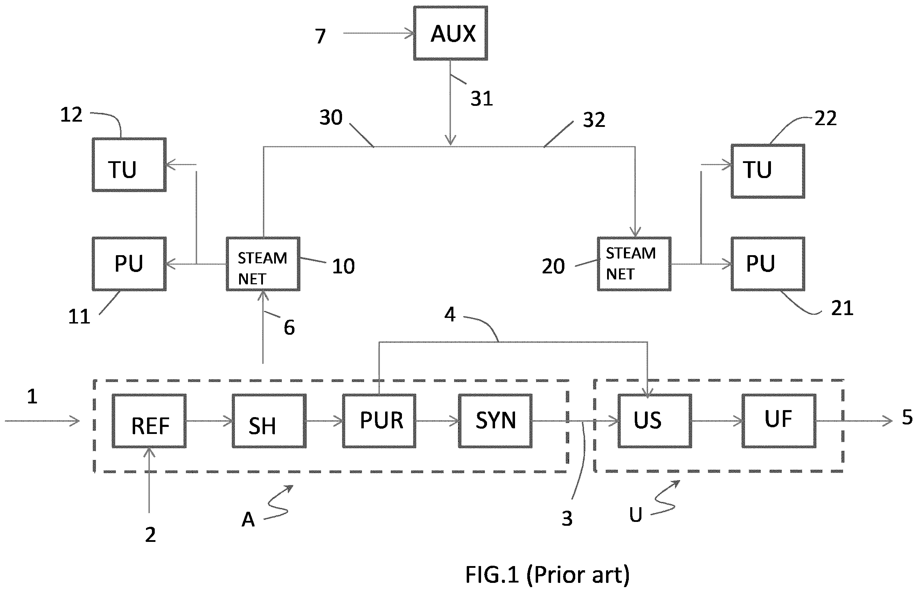

Fig. 1 illustrates a block scheme of a ammonia-urea integrated plant including an ammonia section A and a urea section U. The ammonia section A and urea section U are made according to known art and need not be described in detail.

The ammonia section A includes the following blocks: a reforming section REF, a shift conversion section SH, a syngas purification section PUR, an ammonia synthesis SYN. The blocks REF, SH and PUR are part of a front-end for the generation of ammonia makeup gas.

The urea section U includes the blocks of urea synthesis US and urea finishing UF.

The reforming section REF receives a first input of natural gas 1 as process gas (i.e. natural gas to be reformed) and a second input of natural gas 2 for use as fuel.

The syngas produced in the block REF is processed through blocks SH and PUR and finally reacted in the block SYN to produce ammonia 3.

Said ammonia 3 is reacted in the urea synthesis US together with carbon dioxide 4 removed from the syngas in the block PUR. An input of fresh CO2 may also be provided. The block US produces a urea solution or a urea melt which is processed in the block UF to produce solid urea 5. The solid urea may be obtained by prilling or granulation.

The block 10 denotes the steam network of the ammonia section A. Similarly the block 20 denotes the steam network of the urea section U. The blocks 11, 12 denote the power users and thermal users of the ammonia section A; the blocks 21, 22 denote the power users and thermal users of the urea section U.

The line 6 denotes high grade heat (heat at elevated temperature) which is transferred from the ammonia process to the steam network (e.g. heat removed from hot effluent of steam reforming, etc.).

The ammonia steam network 10 provides steam to the power users 11 (e.g. the syngas compressor) and thermal users 12 (e.g. preheaters, CO2 regeneration). Similarly, the urea steam network 20 provides steam to respective power and thermal users 21, 22.

A medium-pressure steam is typically exported via line 30 from the ammonia steam network 10 to the urea steam network 20, due to excess production of steam in the ammonia section. This steam may be integrated with steam 31 produced in auxiliary boilers AUX to form the steam in line 32 which is actually imported by the urea steam network 20. The steam imported with line 32 satisfies the need of the urea section U for high grade steam, primarily to operate the CO2 compressor and for the high-pressure stripper.

The boilers AUX require an additional amount of fuel 7. Then the fuel input of the process includes the fuel streams 2 and 7.

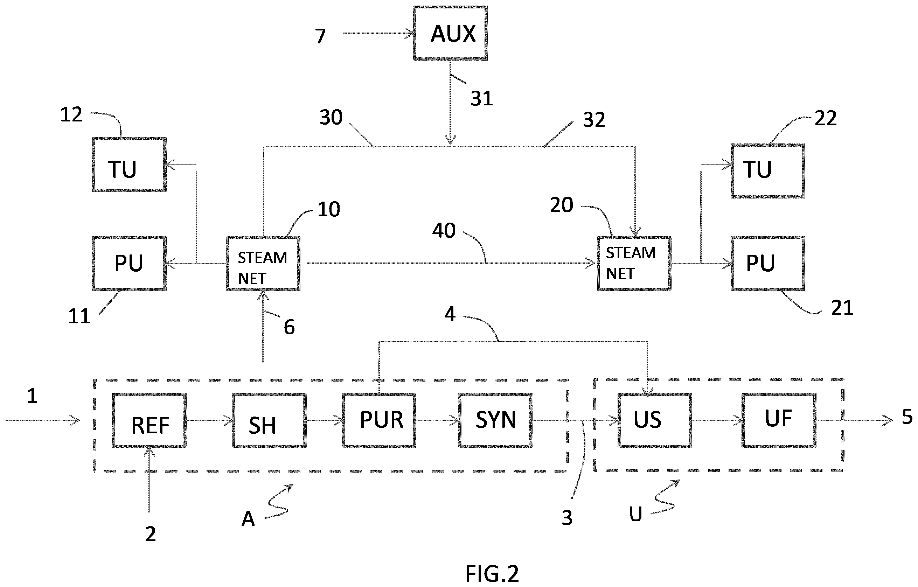

Fig. 2 illustrates, conceptually, the modification of the invention. A low-pressure steam 40 (low-grade heat) is exported from the ammonia steam network 10 to the urea steam network 20. This additional steam 40 is made available thanks to a modernization of the ammonia section A. The urea section U is modified to use some of the heat internally recovered for producing an amount of medium-pressure steam instead of low-pressure. The corresponding deficiency in low-pressure steam in the urea steam network 20 is compensated by the imported steam 40. As a result, the overall consumption (fuels 2 and 7) can be reduced.

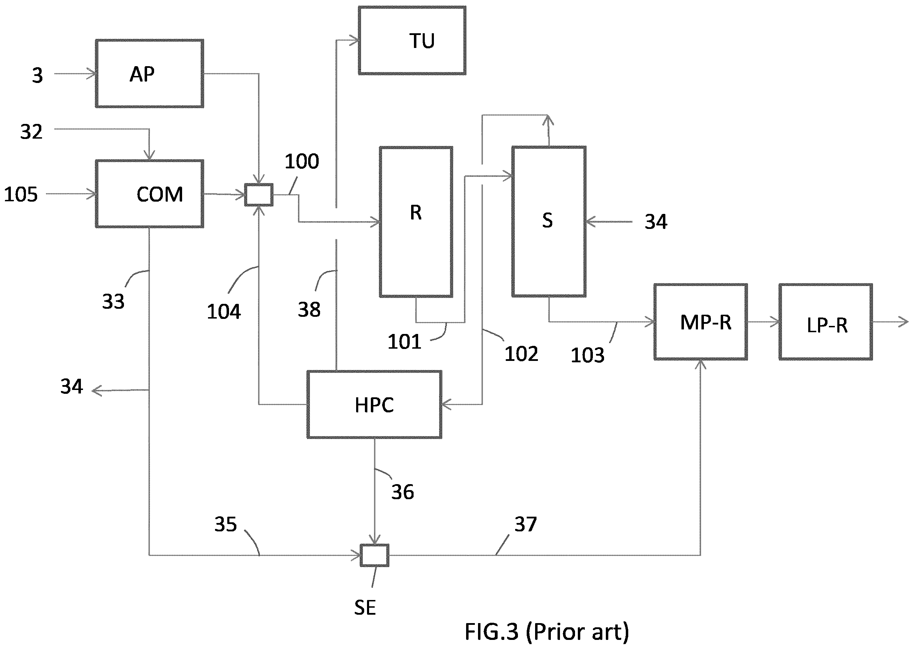

Fig. 3 illustrates a scheme of a urea section U according to the self-stripping concept showing the following items: urea reactor R, high-pressure stripper S, carbamate condenser HPC, ammonia pump AP, CO2 compressor COM, medium-pressure recovery section MP-R and low-pressure recovery section LP-R. The block TU of thermal users is illustrated as a separate block; however it should be understood that some of the thermal users of TU may belong to the recovery section MP-R or LP-R.

The reactor R, stripper S and condenser HPC form a high-pressure synthesis loop. The loop may also include a scrubber for the gas vented from the reactor (not shown). The solution effluent from the stripper S is processed in the recovery section MP-R at medium pressure and then in the section LP-R at low pressure. The effluent of the section LP-R is an aqueous solution of urea consisting mainly of urea and water which may be fed to a finishing section. The unconverted ammonia and carbon dioxide removed in the sections MP-R are returned to the high-pressure synthesis loop normally as a carbamate-containing solution (not shown).

Only some of the connections and process lines are shown in Fig. 3 for simplicity. The main process lines illustrated are the following.
100 reactor input
101 reactor effluent
102 stripper vapours
103 solution effluent from the stripper
104 recycle solution from the HPC
105 input of CO2

Fig. 3 also shows some lines of the steam network. The high grade steam 32 imported from the ammonia section and from the auxiliary boilers is used to operate the CO2 compressor COM particularly to power its steam turbine.

The steam 33 discharged by the turbine is still at medium pressure, for example around 20 bar or higher, and is used partially to heat the stripper S (line 34).

The medium-pressure recovery section MP-R includes a carbamate decomposer which requires steam at around 6 bar, which is a comparatively low pressure but still higher than the pressure of steam that can be produced in the condenser HPC, which is typically around 3.5 bar..

The steam for this carbamate decomposer is therefore produced by mixing part of the MP steam from the turbine, at line 35, with LP steam 36 from the condenser HPC. The resulting steam 37 at the desired pressure of around 6 bar is sent to the decomposer CD. The mixing is performed in a steam ejector SE.

It can be seen that this process involves a let-down of the valuable steam 35 and therefore

A remaining portion of the LP steam produced in the condenser HPC (line 38) is used to heat other thermal users of the urea section.

For example in an embodiment, the MP steam in line 35 has a pressure of about 25 bar; the steam in line 36 has a pressure of about 3.5 bar and the steam in line 37 has a pressure of about 5.5 bar.

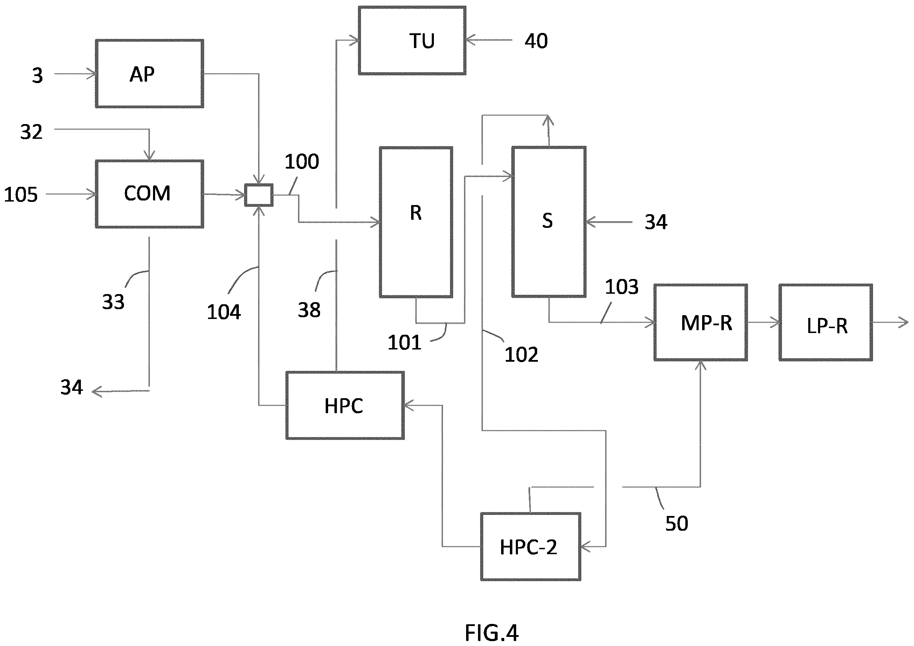

Fig. 4 is an example of implementation of the invention. The urea section U is modified by installing a second condenser HPC-2 in the high-pressure synthesis section. The vapours 102 withdrawn from the stripper S are passed first in the new condenser HPC-2 where a first condensation is performed, and then the effluent of the condenser HPC-2 is further condensed in the original condenser HPC.

The new condenser HPC-2 is also arranged to produce steam at a higher pressure than the original condenser HPC. Particularly, said condenser produces a medium pressure steam 50 which is used to heat the medium-pressure decomposition step in the MP-R section, instead of the stream 37.

As some of the heat contained in the stripper vapours is used to generate said steam 50, the amount of LP steam in the line 38 is reduced. The input of LP steam for the thermal users is therefore balanced with the imported steam 40.

The mixing of medium pressure steam and low pressure steam with the ejector SE is removed. Therefore the process is made more efficient from the point of view of the use of energy. The steam 33 from the turbine of the compression section COM can be entirely destined to the stripper S via line 34.

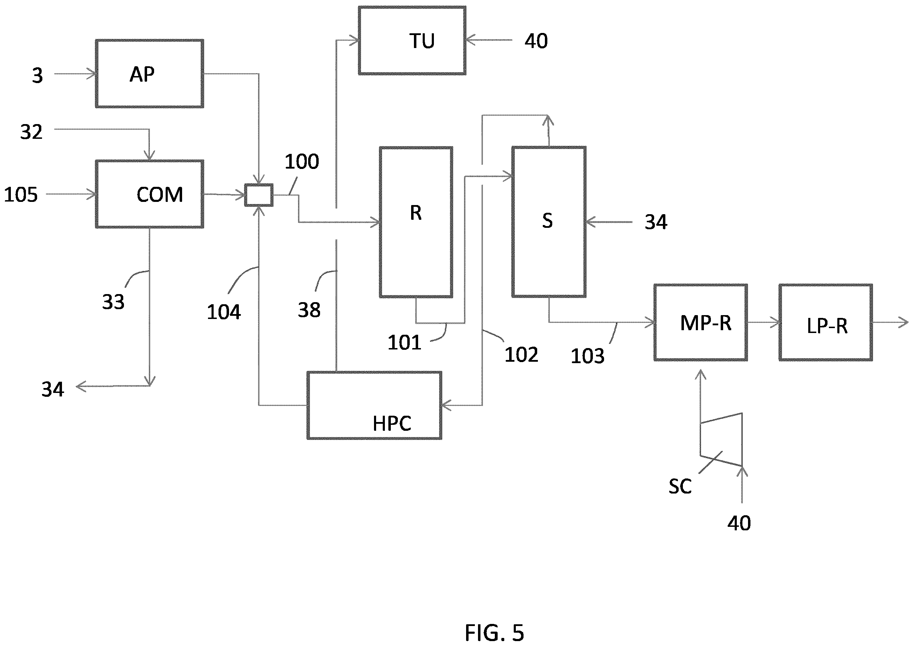

Fig. 5 illustrates another embodiment where steam 40 imported from the ammonia section is raised to the pressure required by the section MP-R in a steam compressor SC. For example the steam 40 is raised from 3.5 bar to 5.5 bar. Also in this embodiment the inefficient mixing in the ejector SE can be removed. Part of the steam 40 may also be used in the users TU.

The embodiments of Fig. 4 with double-pressure condensation and of Fig. 5 with compression of steam are usually alternative embodiments, however they may be combined if appropriate.

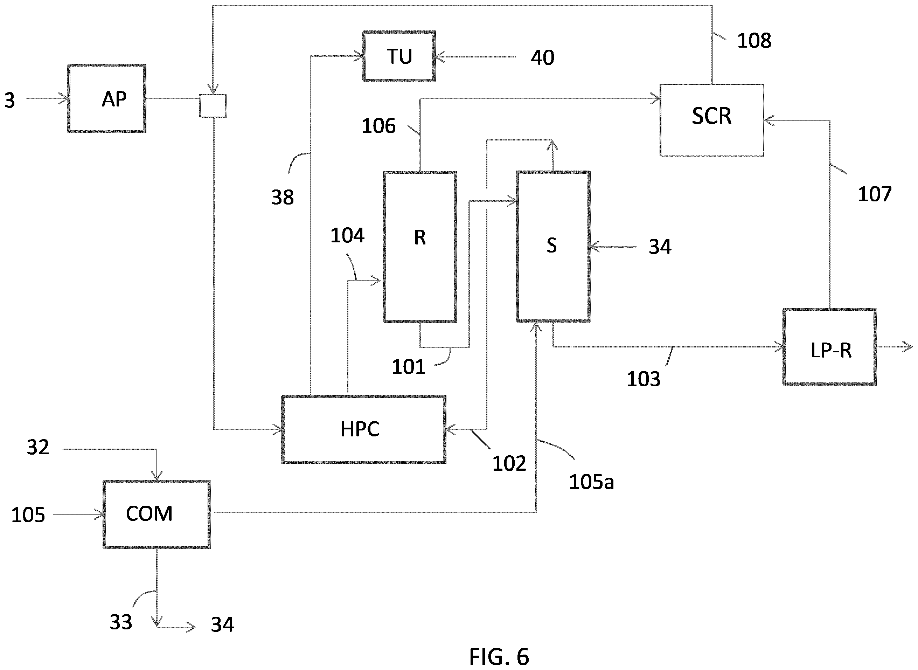

Fig. 6 illustrates a scheme of a CO2 stripping urea section. The carbon dioxide delivered by the compression section COM is introduced in the synthesis loop via the stripper S, where it acts as a stripping aid (line 105a). The synthesis loop typically comprises also a high-pressure scrubber SCR where gas 106 vented from the reactor are scrubbed with a recycle solution 107 coming from the low-pressure recovery section LP-R. The fresh ammonia enters the loop via the condenser HPC, together with recycle solution 108 from the scrubber.

The recovery is performed only at a low pressure in the LP-R section. Therefore the urea section normally has no user of MLP steam like the MP carbamate decomposer previously described.

In such a case a preferred embodiment of the invention provides that the duty of the stripper S is reduced and shifted to the LP-R section. To some extent, this can be made without affecting the performance of the reactor R. Accordingly the steam 34 required by the stripper S is reduced, which is a saving of energy. The condenser HPC as a consequence has also a reduced duty and can produce less steam 38; the missing steam is compensated by the import 40 from the ammonia section.

## Claims

1. A method of revamping an ammonia-urea plant wherein:
A) the ammonia-urea plant which is revamped has the following features:
the ammonia urea plant includes an ammonia section and a urea section,
said ammonia section includes a front-end for generation of a makeup syngas from reforming of a hydrocarbon and an ammonia synthesis section where the makeup syngas is reacted to produce ammonia
ammonia produced in the ammonia synthesis section, and at least part of the CO2 removed from the makeup syngas in the front-end, are fed to the urea section for use as starting material for the production of urea,
the urea section includes a high-pressure urea synthesis section including at least a urea synthesis reactor, a stripper for removing unconverted ammonia and carbon dioxide from the reactor effluent, and a condensation stage arranged to condensate stripper vapours containing ammonia and carbon dioxide removed from the reactor effluent,
the urea section further includes a recovery section for recovering unconverted ammonia and carbon dioxide from the solution effluent from the stripper, at a pressure lower than the urea synthesis pressure,
the ammonia-urea plant also includes a steam network, including steam producers and steam users, and steam produced in the ammonia section is exported to the urea section and sent to at least one steam user in the urea section,
the condensation stage of the high-pressure urea synthesis section is a steam producer in the steam network and produces low pressure steam at a first pressure using the heat of condensation of the stripper vapours, said low pressure steam being used internally in the urea section in one or more steam users of the urea section,
and
B) the revamping includes:
the provision of an export of low pressure steam at a pressure not greater than 6 bar rel, preferably 2 to 6 bar rel, from the ammonia section to the urea section, for use in at least one steam user in the urea section.

2. A method according to claim 1 wherein the urea section is modified to accommodate said low pressure steam exported from the ammonia section to the urea section.

3. A method according to claim 2 wherein:
the urea section includes a steam user of steam at a second pressure greater than the pressure of said low pressure steam transferred from the ammonia section to the urea section;
the method includes modifying the urea section to produce steam at said second pressure for said user and balancing the input of low-pressure steam to the urea section with said low-pressure steam transferred from the ammonia section to the urea section.

4. A method according to claim 3 wherein the step of modifying the urea section to produce steam at said second pressure includes modifying the condensation stage of the high-pressure urea synthesis section to use part of the condensation heat of the stripper vapours to produce said steam at said second pressure.

5. A method according to claim 4, wherein the condensation stage of the urea synthesis section originally includes a first condenser arranged to produce low-pressure steam at said first pressure, and the revamping of the stage includes the installation of a second condenser arranged to produce said medium-pressure steam at said second pressure.

6. A method according to claim 5 wherein the second condenser is installed in series and upstream the first condenser, so that the stripper vapours coming from the stripper pass through the newly installed second condenser and then through the original first condenser.

7. A method according to claim 5 or 6, wherein the first condenser and/or the second condenser are shell-and-tube equipment, preferably horizontally arranged of the kettle type, where the stripper vapours flow in the tube side and steam is produced in the shell side.

8. A method according to any of claims 3 to 7 wherein the step of modifying the urea section to produce steam at said second pressure includes installation of a steam compressor which is fed with steam at low pressure and delivers steam at said second pressure.

9. A method according to any of claims 3 to 8 wherein:
in the ammonia-urea plant, the steam at said second pressure for said user is originally produced by mixing medium-pressure steam with low-pressure steam produced in the condensation stage,
the method includes that said mixing is discontinued

10. A method according to any of claims 3 to 9, wherein the urea section includes a medium-pressure recovery section and a low-pressure recovery section and said steam user of said steam at the second pressure is a carbamate decomposer of the medium-pressure recovery section.

11. A method according to claim 10 wherein the urea section operates with ammonia-stripping or self-stripping process.

12. A method according to any of claims 3 to 11 wherein said second pressure is 5 bar to 7 bar, preferably 6 bar or around 6 bar.

13. A method according to claim 2 wherein:
the urea section is a CO2-stripping section wherein the recovery section includes only a section at low pressure;
the method includes reducing the amount of heat transferred to the stripper thus reducing also the amount of low pressure steam that can be produced in the high-pressure carbamate condenser, and
the missing production of low pressure steam in the urea section is balanced with said low-pressure steam transferred from the ammonia section to the urea section.

14. A method according to any of the previous claims, wherein the ammonia section is modified to increase the low pressure steam that can be exported form the ammonia section and therefore to provide said low pressure steam for transfer to the urea section, preferably as a result of increase efficiency of LP steam utilization or as a result of increased heat recovery.

15. A method according to claim 14, wherein at least part of the low pressure steam for balancing the input of the urea section is obtained from modernization of a CO2 recovery section in the front-end of the ammonia section, such modernization resulting in a reduced specific consumption of regeneration energy for the CO2 recovery section, preferably down to a specific consumption of less than 400 kcal/Nm3 of removed CO2.

16. A method according to any of the previous claims, further including that the steam to carbon ratio in the reforming process is lowered, and preferably including that the ammonia section is revamped to operate the reforming process with a S/C ratio of less than 2.7, more preferably in the range of 2.6 to 2.0.

17. A method according to claim 16 wherein the shift section is modified to operate with said reduced S/C ratio and/or wherein at least one steam turbine driver is changed to a motor drive resulting in reduced steam consumption.

18. An integrated ammonia-urea process comprising:
ammonia is produced in an ammonia section by generation of a makeup syngas from reforming of a hydrocarbon and reaction of said makeup syngas to produce ammonia
at least part of the synthesized ammonia and optionally also CO2 removed from the makeup syngas during its purification are used in a tied-in urea section for the production of urea,
wherein urea is produced with a stripping process at high synthesis pressure and includes recovery of unconverted matter at one or more pressure levels lower than said synthesis pressure, ,
wherein steam is produced internally in both the ammonia section and the urea section for use in steam users of the plant,
the process being **characterized in that:**
a low pressure steam at a pressure of pressure not greater than 6 bar rel, preferably 2 to 6 bar rel, is transferred from the ammonia section to the urea section, for use in at least one steam user in the urea section.

19. A process according to claim 18, wherein:
urea is produced with a self-stripping or with an ammonia-stripping process and recovery is performed at two pressure levels, namely at a medium pressure and at a low pressure,
said steam transferred from the ammonia section to the urea section is used for carbamate decomposition in the medium-pressure recovery,
or
urea is produced with a CO2-stripping process and recovery is performed at a single pressure level, namely at a low pressure,
said steam transferred from the ammonia section to the urea section is used together with steam produced in the carbamate condenser of the synthesis section, to provide steam for low-pressure steam users of the urea section.

## Patentansprüche

1. Verfahren zur Nachrüstung einer Ammoniak-Harnstoff-Anlage, wobei:
A) die Ammoniak-Harnstoff-Anlage, die nachgerüstet wird, die folgenden Merkmale aufweist:
die Ammoniak-Harnstoff-Anlage umfasst einen Ammoniakabschnitt und einen Harnstoffabschnitt, wobei der Ammoniakabschnitt ein Front-End zur Erzeugung eines Make-up-Synthesegases aus der Reformierung eines Kohlenwasserstoffs und einen Ammoniaksyntheseabschnitt umfasst, in dem das Make-up-Synthesegas umgesetzt wird, um in dem Ammoniak-Syntheseabschnitt erzeugtes Ammoniak-Ammoniak zu produzieren, und zumindest ein Teil des aus dem Make-up-Synthesegas in dem Front-End entfernten CO₂ dem Harnstoffabschnitt zur Verwendung als Ausgangsmaterial für die Herstellung von Harnstoff zugeführt werden, wobei der Harnstoffabschnitt einen Hochdruck-Harnstoffsyntheseabschnitt umfasst, der zumindest einen Harnstoffsynthesereaktor, einen Stripper zum Entfernen von nicht umgewandeltem Ammoniak und Kohlendioxid aus dem Reaktorausfluss und eine Kondensationsstufe umfasst, die so angeordnet ist, dass sie Stripperdämpfe kondensiert, die aus dem Reaktorausfluss entferntes Ammoniak und Kohlendioxid enthalten, der Harnstoffabschnitt ferner einen Rückgewinnungsabschnitt zur Rückgewinnung von nicht umgewandeltem Ammoniak und Kohlendioxid aus der aus dem Stripper ausströmenden Lösung bei einem Druck, der niedriger ist als der Harnstoffsynthesedruck, umfasst, die Ammoniak-Harnstoff-Anlage auch ein Dampfnetz mit Dampferzeugern und Dampfverbrauchern umfasst und der im Ammoniakabschnitt erzeugte Dampf in den Harnstoffabschnitt exportiert und zu mindestens einem Dampfverbraucher im Harnstoffabschnitt geleitet wird, die Kondensationsstufe des Hochdruck-Harnstoffsyntheseabschnitts ein Dampferzeuger im Dampfnetz ist und unter Nutzung der Kondensationswärme der Stripperdämpfe Niederdruckdampf mit einem ersten Druck erzeugt, wobei der Niederdruckdampf intern im Harnstoffabschnitt in einem oder mehreren Dampfverbrauchern des Harnstoffabschnitts verwendet wird, und
B) die Nachrüstung umfasst weiter:
die Bereitstellung eines Exports von Niederdruckdampf mit einem Druck von nicht mehr als 6 bar rel, vorzugsweise 2 bis 6 bar rel, vom Ammoniakabschnitt zum Harnstoffabschnitt zur Verwendung in mindestens einem Dampfverbraucher im Harnstoffabschnitt.

2. Verfahren nach Anspruch 1, wobei der Harnstoffabschnitt so modifiziert wird, dass er den aus dem Ammoniakabschnitt in den Harnstoffabschnitt geleiteten Niederdruckdampf aufnehmen kann.

3. Verfahren nach Anspruch 2, wobei: der Harnstoffabschnitt einen Dampfverbraucher von Dampf mit einem zweiten Druck enthält, der größer ist als der Druck des Niederdruckdampfes, der vom Ammoniakabschnitt zum Harnstoffabschnitt übertragen wird; wobei das Verfahren das Modifizieren des Harnstoffabschnitts umfasst, um Dampf mit dem zweiten Druck für den Verbraucher zu erzeugen und die Zufuhr von Niederdruckdampf zum Harnstoffabschnitt mit dem Niederdruckdampf auszugleichen, der vom Ammoniakabschnitt zum Harnstoffabschnitt übertragen wird.

4. Verfahren nach Anspruch 3, wobei der Schritt des Modifizierens des Harnstoffabschnitts zur Erzeugung von Dampf bei dem zweiten Druck das Modifizieren der Kondensationsstufe des Hochdruck-Harnstoffsyntheseabschnitts umfasst, um einen Teil der Kondensationswärme der Stripperdämpfe zur Erzeugung des Dampfs bei dem zweiten Druck zu nutzen.

5. Verfahren nach Anspruch 4, wobei die Kondensationsstufe des Harnstoffsyntheseabschnitts ursprünglich einen ersten Kondensator umfasst, der so angeordnet ist, dass er Niederdruckdampf bei dem ersten Druck erzeugt, und die Nachrüstung der Stufe die Installation eines zweiten Kondensators umfasst, der so angeordnet ist, dass er den Mitteldruckdampf bei dem zweiten Druck erzeugt.

6. Verfahren nach Anspruch 5, bei dem der zweite Kondensator in Reihe und stromaufwärts des ersten Kondensators installiert wird, so dass die vom Stripper kommenden Stripperdämpfe durch den neu installierten zweiten Kondensator und dann durch den ursprünglichen ersten Kondensator strömen.

7. Verfahren nach Anspruch 5 oder 6, wobei der erste Kondensator und/oder der zweite Kondensator eine Rohrbündelanlage, vorzugsweise eine horizontal angeordnete Kesselanlage, ist, bei der die Stripperdämpfe in der Rohrseite strömen und der Dampf in der Mantelseite erzeugt wird.

8. Verfahren nach einem der Ansprüche 3 bis 7, wobei der Schritt des Modifizierens des Harnstoffabschnitts zur Erzeugung von Dampf bei dem zweiten Druck die Installation eines Dampfkompressors umfasst, der mit Dampf bei niedrigem Druck gespeist wird und Dampf bei dem zweiten Druck liefert.

9. Verfahren nach einem der Ansprüche 3 bis 8, wobei: in der Ammoniak-Harnstoff-Anlage der Dampf mit dem zweiten Druck für den Benutzer ursprünglich durch Mischen von Mitteldruckdampf mit Niederdruckdampf, der in der Kondensationsstufe erzeugt wird, hergestellt wird, wobei das Verfahren beinhaltet, dass das Mischen unterbrochen wird.

10. Verfahren nach einem der Ansprüche 3 bis 9, wobei der Harnstoffabschnitt einen Mitteldruck-Rückgewinnungsabschnitt und einen Niederdruck-Rückgewinnungsabschnitt umfasst und der Dampfverbraucher des Dampfes bei dem zweiten Druck ein Carbamatzersetzer des Mitteldruck-Rückgewinnungsabschnitts ist.

11. Verfahren nach Anspruch 10, wobei der Harnstoffabschnitt mit dem Ammoniak-Stripping- oder Selbst-Stripping-Verfahren arbeitet.

12. Verfahren nach einem der Ansprüche 3 bis 11, wobei der zweite Druck 5 bar bis 7 bar, vorzugsweise 6 bar oder etwa 6 bar beträgt.

13. Verfahren nach Anspruch 2, wobei: der Harnstoffabschnitt ein CO₂-Stripper-Abschnitt ist, wobei der Rückgewinnungsabschnitt nur einen Abschnitt mit niedrigem Druck umfasst; das Verfahren die Verringerung der an den Stripper übertragenen Wärmemenge umfasst, wodurch auch die Menge an Niederdruckdampf verringert wird, die in dem Hochdruck-Carbamatkondensator erzeugt werden kann, und die fehlende Erzeugung von Niederdruckdampf in dem Harnstoffabschnitt mit dem von dem Ammoniakabschnitt zu dem Harnstoffabschnitt übertragenen Niederdruckdampf ausgeglichen wird.

14. Verfahren nach einem der vorhergehenden Ansprüche, wobei der Ammoniakabschnitt modifiziert wird, um den Niederdruckdampf zu erhöhen, der aus dem Ammoniakabschnitt exportiert werden kann, und daher den Niederdruckdampf für die Übertragung auf den Harnstoffabschnitt bereitzustellen, vorzugsweise als Ergebnis einer erhöhten Effizienz der Niederdruckdampfnutzung oder als Ergebnis einer erhöhten Wärmerückgewinnung.

15. Verfahren nach Anspruch 14, bei dem zumindest ein Teil des Niederdruckdampfes zum Ausgleich des Eingangs des Harnstoffabschnitts aus der Modernisierung eines CO₂-Rückgewinnungsabschnitts am vorderen Ende des Ammoniakabschnitts gewonnen wird, wobei eine solche Modernisierung zu einem verringerten spezifischen Verbrauch an Regenerationsenergie für den CO₂-Rückgewinnungsabschnitt führt, vorzugsweise bis zu einem spezifischen Verbrauch von weniger als 400 kcal/Nm³ des entfernten CO₂.

16. Verfahren nach einem der vorhergehenden Ansprüche, wobei ferner das Dampf-Kohlenstoff-Verhältnis im Reformierungsprozess gesenkt wird, und vorzugsweise der Ammoniakabschnitt so umgestaltet wird, dass der Reformierungsprozess mit einem Sie-Verhältnis von weniger als 2,7, vorzugsweise im Bereich von 2,6 bis 2,0, betrieben wird.

17. Verfahren nach Anspruch 16, bei dem der Schaltabschnitt so modifiziert wird, dass er mit dem reduzierten Sie-Verhältnis arbeitet, und/oder bei dem zumindest ein Dampfturbinenantrieb auf einen Motorantrieb umgestellt wird, was zu einem reduzierten Dampfverbrauch führt.

18. Ein integriertes Ammoniak-Harnstoff-Verfahren, das Folgendes umfasst: Ammoniak in einem Ammoniakabschnitt durch Erzeugung eines Synthesegases aus der Reformierung eines Kohlenwasserstoffs und Reaktion des Synthesegases zur Herstellung von Ammoniak hergestellt wird, zumindest ein Teil des synthetisierten Ammoniaks und gegebenenfalls auch CO₂, das aus dem Synthesegas während seiner Reinigung entfernt wird, in einem eingebundenen Harnstoffabschnitt zur Herstellung von Harnstoff verwendet werden, wobei Harnstoff mit einem Strippverfahren bei hohem Synthesedruck hergestellt wird und die Rückgewinnung von nicht umgewandeltem Material bei einem oder mehreren Druckniveaus, die niedriger als der Synthesedruck sind, einschließt, wobei Dampf intern sowohl in dem Ammoniakabschnitt als auch in dem Harnstoffabschnitt zur Verwendung in Dampfverbrauchern der Anlage erzeugt wird, wobei das Verfahren **dadurch gekennzeichnet ist, dass**: ein Niederdruckdampf mit einem Druck von nicht mehr als 6 bar rel, vorzugsweise 2 bis 6 bar rel, von dem Ammoniakabschnitt zu dem Harnstoffabschnitt übertragen wird, um in mindestens einem Dampfverbraucher in dem Harnstoffabschnitt verwendet zu werden.

19. Verfahren nach Anspruch 18, wobei: Harnstoff mit einem Self-Stripping- oder mit einem Ammoniak-Stripping-Verfahren hergestellt wird und die Rückgewinnung auf zwei Druckniveaus erfolgt, nämlich bei einem mittleren Druck und bei einem niedrigen Druck, wobei der von dem Ammoniakabschnitt zu dem Harnstoffabschnitt übertragene Dampf für die Carbamatzersetzung in der Mitteldruckrückgewinnung verwendet wird, oder Harnstoff mit einem CO₂-Stripping-Verfahren hergestellt wird und die Rückgewinnung auf einem einzigen Druckniveau, nämlich bei einem niedrigen Druck, erfolgt, wobei der vom Ammoniakabschnitt in den Harnstoffabschnitt übertragene Dampf zusammen mit dem im Carbamatkondensator des Syntheseabschnitts erzeugten Dampf verwendet wird, um Dampf für Niederdruck-Dampfverbraucher des Harnstoffabschnitts bereitzustellen.

## Revendications

1. Procédé de modernisation d'une installation du type ammoniac-urée dans lequel :
A) l'installation du type ammoniac-urée qui est modernisée présente les caractéristiques suivantes :
l'installation du type ammoniac-urée inclut une section d'ammoniac et une section d'urée,
ladite section d'ammoniac inclut une extrémité avant pour une génération d'un gaz de synthèse d'appoint à partir du reformage d'un hydrocarbure et une section de synthèse d'ammoniac où le gaz de synthèse d'appoint est amené à réagir pour produire de l'ammoniac
l'ammoniac produit dans la section de synthèse d'ammoniac, et au moins une partie du CO2 éliminé du gaz de synthèse d'appoint dans l'extrémité avant, sont introduits dans la section d'urée pour être utilisés comme matière de base pour la production d'urée,
la section d'urée inclut une section de synthèse d'urée à haute pression incluant au moins un réacteur de synthèse d'urée, un rectificateur destiné à éliminer l'ammoniac et le dioxyde de carbone non convertis de l'effluent de réacteur, et un étage de condensation conçu pour condenser des vapeurs de rectificateur contenant de l'ammoniac et du dioxyde de carbone éliminés de l'effluent de réacteur,
la section d'urée inclut en outre une section de récupération destinée à récupérer l'ammoniac et le dioxyde de carbone non convertis de l'effluent de solution à partir du rectificateur, à une pression inférieure à la pression de synthèse d'urée,
l'installation du type ammoniac-urée inclut un réseau de vapeur, incluant des producteurs de vapeur et des utilisateurs de vapeur, et une vapeur produite dans la section d'ammoniac est exportée vers la section d'urée et envoyée à au moins un utilisateur de vapeur dans la section d'urée,
l'étage de condensation de la section de synthèse d'urée à haute pression est un producteur de vapeur dans le réseau de vapeur et produit une vapeur à basse pression à une première pression au moyen de la chaleur de condensation des vapeurs de rectificateur, ladite vapeur à basse pression étant utilisée en interne dans la section d'urée dans un ou plusieurs utilisateurs de vapeur de la section d'urée,
et
B) la modernisation inclut :
la fourniture d'un export de vapeur à basse pression à une pression non supérieure à 6 bars rel, de préférence de 2 à 6 bars rel, de la section d'ammoniac à la section d'urée, destiné à être utilisé dans au moins un utilisateur de vapeur dans la section d'urée.

2. Procédé selon la revendication 1 dans lequel la section d'urée est modifiée pour recevoir ladite vapeur à basse pression exportée de la section d'ammoniac à la section d'urée.

3. Procédé selon la revendication 2 dans lequel :
la section d'urée inclut un utilisateur de vapeur de vapeur à une seconde pression supérieure à la pression de ladite vapeur à basse pression transférée de la section d'ammoniac à la section d'urée ;
le procédé inclut la modification de la section d'urée pour produire une vapeur à ladite seconde pression pour ledit utilisateur et l'équilibrage de l'entrée de la vapeur à basse pression dans la section d'urée avec ladite vapeur à basse pression transférée de la section d'ammoniac à la section d'urée.

4. Procédé selon la revendication 3 dans lequel l'étape de modification de la section d'urée pour produire une vapeur à ladite seconde pression inclut la modification de l'étage de condensation de la section de synthèse d'urée à haute pression pour utiliser une partie de la chaleur de condensation des vapeurs de rectificateur pour produire ladite vapeur à ladite seconde pression.

5. Procédé selon la revendication 4, dans lequel l'étage de condensation de la section de synthèse d'urée inclut à l'origine un premier condenseur conçu pour produire une vapeur à basse pression à ladite première pression, et la modernisation de l'étage inclut l'installation d'un second condenseur conçu pour produire ladite vapeur à moyenne pression à ladite seconde pression.

6. Procédé selon la revendication 5 dans lequel le second condenseur est installé en série et en amont du premier condenseur, de sorte que les vapeurs de rectificateur provenant du rectificateur traversent le second condenseur nouvellement installé, puis le premier condenseur d'origine.

7. Procédé selon la revendication 5 ou 6, dans lequel le premier condenseur et/ou le second condenseur sont un équipement à enveloppe et tube, de préférence horizontalement agencés du type chaudière, où les vapeurs de rectificateur s'écoulent côté tube et une vapeur est produite côté enveloppe.

8. Procédé selon l'une quelconque des revendications 3 à 7 dans lequel l'étape de modification de la section d'urée pour produire une vapeur à ladite seconde pression inclut l'installation d'un compresseur de vapeur qui est alimenté en vapeur à basse pression et distribue de la vapeur à ladite seconde pression.

9. Procédé selon l'une quelconque des revendications 3 à 8 dans lequel :
dans l'installation du type ammoniac-urée, la vapeur à ladite seconde pression pour ledit utilisateur est produite à l'origine en mélangeant une vapeur à moyenne pression avec une vapeur à basse pression produite dans l'étage de condensation,
le procédé inclut que ledit mélange est interrompu.

10. Procédé selon l'une quelconque des revendications 3 à 9, dans lequel la section d'urée inclut une section de récupération à moyenne pression et une section de récupération à basse pression et ledit utilisateur de vapeur de ladite vapeur à la seconde pression est un décomposeur de carbamate de la section de récupération à moyenne pression.

11. Procédé selon la revendication 10 dans lequel la section d'urée opère avec un processus de rectification d'ammoniac ou de rectification automatique.

12. Procédé selon l'une quelconque des revendications 3 à 11 dans lequel ladite seconde pression est de 5 bars à 7 bars, de préférence de 6 bars ou d'environ 6 bars.

13. Procédé selon la revendication 2 dans lequel :
la section d'urée est une section de rectification de CO2 dans laquelle la section de récupération inclut seulement une section à basse pression ;
le procédé inclut la réduction de la quantité de chaleur transférée au rectificateur réduisant ainsi la quantité de vapeur à basse pression qui peut être produite dans le condenseur de carbamate à haute pression, et
la production manquante de vapeur à basse pression dans la section d'urée est équilibrée avec ladite vapeur à basse pression transférée de la section d'ammoniac à la section d'urée.

14. Procédé selon l'une quelconque des revendications précédentes, dans lequel la section d'ammoniac est modifiée pour augmenter la vapeur à basse pression qui peut être exportée à partir de la section d'ammoniac et pour fournir ainsi ladite vapeur à basse pression pour le transfert à la section d'urée, de préférence en raison d'une efficacité d'augmentation d'une utilisation de vapeur à basse pression ou en raison d'une récupération de chaleur accrue.

15. Procédé selon la revendication 14, dans lequel au moins une partie de la vapeur à basse pression pour l'équilibrage de l'entrée de la section d'urée est obtenue à partir d'une modernisation d'une section de récupération de CO2 dans l'extrémité avant de la section d'ammoniac, ladite modernisation résultant en une réduction de consommation spécifique d'énergie de récupération pour la section de récupération de CO2, de préférence jusqu'à obtenir une consommation spécifique de moins de 400 kcal/Nm3 de CO2 éliminé.

16. Procédé selon l'une quelconque des revendications précédentes, incluant en outre que le rapport vapeur/carbone dans le processus de reformage est abaissé, et incluant de préférence que la section d'ammoniac est modernisée pour opérer le processus de reformage avec un rapport V/C de moins de 2,7, de préférence encore dans la plage de 2,6 à 2,0.

17. Procédé selon la revendication 16 dans lequel la section de décalage est modifiée pour opérer avec ledit rapport V/C réduit et/ou dans lequel au moins un entraîneur de turbine à vapeur est transformé en un entraînement de moteur résultant en une consommation de vapeur réduite.

18. Processus du type ammoniac-urée intégré comprenant :
l'ammoniac est produit dans une section d'ammoniac par la génération d'un gaz de synthèse d'appoint à partir du reformage d'un hydrocarbure et de la réaction dudit gaz de synthèse d'appoint pour produire de l'ammoniac
au moins une partie de l'ammoniac synthétisé et éventuellement également de CO2 éliminé à partir du gaz de synthèse d'appoint pendant sa purification sont utilisés dans une section d'urée liée pour la production d'urée,
dans lequel de l'urée est produite avec un processus de rectification à haute pression de synthèse et inclut la récupération de matière non convertie à un ou plusieurs niveaux de pression inférieurs à ladite pression de synthèse,
dans lequel de la vapeur est produite en interne à la fois dans la section d'ammoniac et la section d'urée pour une utilisation dans des utilisateurs de vapeur de l'installation,
le processus étant **caractérisé en ce que** :
une vapeur à basse pression à une pression de pression non supérieure à 6 bars rel, de préférence de 2 à 6 bars rel, est transférée de la section d'ammoniac à la section d'urée, pour une utilisation dans au moins un utilisateur de turbine dans la section d'urée.

19. Processus selon la revendication 18, dans lequel :
de l'urée est produite avec un processus de rectification automatique ou un processus de rectification d'ammoniac et une récupération est réalisée à deux niveaux de pression, à savoir à une moyenne pression et à une basse pression,
ladite vapeur transférée de la section d'ammoniac à la section d'urée est utilisée pour une décomposition de carbamate dans la récupération à moyenne pression,
ou
de l'urée est produite avec un processus de rectification de CO2 et une récupération est réalisée à un niveau de pression unique, à savoir à une basse pression,
ladite vapeur transférée de la section d'ammoniac à la section d'urée est utilisée conjointement avec une vapeur produite dans le condenseur de carbamate de la section de synthèse, pour fournir une vapeur pour des utilisateurs de vapeur à basse pression de la section d'urée.
